(19) Europäisches Patentamt
European Patent Office
Office européen des brevets

(11) EP 0 562 061 B1

(12) **EUROPÄISCHE PATENTSCHRIFT**

(45) Veröffentlichungstag und Bekanntmachung des Hinweises auf die Patenterteilung:
**22.01.1997 Patentblatt 1997/04**

(21) Anmeldenummer: **92917565.1**

(22) Anmeldetag: **19.08.1992**

(51) Int. Cl.$^6$: **A61K 7/13**, C07D 487/04

(86) Internationale Anmeldenummer:
**PCT/EP92/01887**

(87) Internationale Veröffentlichungsnummer:
**WO 93/07849 (29.04.1993 Gazette 1993/11)**

(54) **HAARFÄRBEMITTEL MIT EINEM GEHALT AN AMINOPYRAZOLDERIVATEN SOWIE NEUE PYRAZOLDERIVATE**

HAIR-DYEING AGENT CONTAINING AMINOPYRAZOLE DERIVATIVES, AND NEW PYRAZOLE DERIVATIVES

COLORANT POUR LA TEINTURE DES CHEVEUX RENFERMANT DES DERIVES AMINOPYRAZOLIQUES ET NOUVEAUX DERIVES PYRAZOLIQUES

(84) Benannte Vertragsstaaten:
**DE ES FR GB IT**

(30) Priorität: **14.10.1991 DE 4133957**

(43) Veröffentlichungstag der Anmeldung:
**29.09.1993 Patentblatt 1993/39**

(73) Patentinhaber: **Wella Aktiengesellschaft**
**64295 Darmstadt (DE)**

(72) Erfinder:
- **NEUNHOEFFER, Hans**
  **D-6109 Mühltal (DE)**
- **GERSTUNG, Stefan**
  **D-6107 Reinheim (DE)**
- **CLAUSEN, Thomas**
  **D-6146 Alsbach (DE)**
- **BALZER, Wolfgang, R.**
  **D-6146 Alsbach (DE)**

(56) Entgegenhaltungen:
**EP-A- 0 264 773** **EP-A- 0 433 854**
**WO-A-92/04883**

- **ANTI-CANCER DRUG DES. Bd. 2, Nr. 3, 1987, GB Seiten 235 - 245 GODDARD A. ,ORR**
- **R. ,STOCK J. 'synthesis and ribonucleotide reductase inhibitory activity of analogs of 2,3-dihydro-1 h-imidazo(1,2-b)pyrazole (impy)'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.13/3.4

## Beschreibung

Gegenstand der Erfindung sind Mittel zur oxidativen Färbung von Keratinfasern auf Basis von Aminopyrazolderivaten sowie neue Pyrazolderivate.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, 5-Amino-2-methylphenol und Derivate des m-Phenylendiamins zu nennen.

An Oxidationsfarbstoffe, die zur Färbung menschlicher Haare verwendet werden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombinationen geeigneter Entwickler- und Kupplerkomponenten eine breite Palette verschiedener Farbnuancen erzeugt werden kann. Zur Erzielung natürlicher und insbesondere modischer Nuancen im Rotbereich wird vor allem 4-Aminophenol, allein oder im Gemisch mit anderen Entwicklersubstanzen, in Kombination mit geeigneten Kupplersubstanzen eingesetzt.

Gegen den für den Rotbereich der Farbskala bisher hauptsächlich eingesetzten Entwickler 4-Aminophenol wurden in letzter Zeit Bedenken in bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können. Die in der DE-OS 21 60 317 beschriebenen Pyrazolderivate, wie zum Beispiel das 3-Amino-1-phenyl-2-pyrazolon-5, färben Haare nur in sehr geringen, für die Haarfärbepraxis unbrauchbaren, Farbtiefen an.

Die in der DE-OS 38 43 893 beschriebenen 4,5-Diaminopyrazole erfüllen zwar die im Hinblick auf Farbintensität geforderten Ansprüche, besitzen aber noch Nachteile, die eine Verwendung als Haarfarbstoffe erschweren. So zeigt z. B. das 3(5),4-Diaminopyrazol ein leicht sensibilisierendes Potential. Andere Verbindungen, wie das 4,5-Diamino-1-methyl-pyrazol oder das 4,5-Diamino-1-benzyl-pyrazol sind nur aufwendig herzustellen, da im Verlauf der Synthese Isomerengemische anfallen, welche chromatographisch aufgetrennt werden müssen.

Aus Anti-Cancer Drug Design (1987) **2**, Seiten 235 - 245 ist ein 2,3-Dihydro-7-nitro-1H-imidazo-[1,2-b]-pyrazol bekannt.

Es bestand daher die Aufgabe, ein Oxidationshaarfärbemittel auf der Basis einer Entwicklersubstanz-Kupplersubstanz-Kombination zur Verfügung zu stellen, in dem eine Entwicklersubstanz für den Rotbereich enthalten ist, welche physiologisch gut verträglich ist, einfach herzustellen ist und welche mit üblichen Kupplersubstanzen das Haar in brillanten roten Farbtönen mit einer hohen Farbtiefe färbt.

Hierzu wurde nun gefunden, daß durch ein Mittel zur oxidativen Färbung von Haaren auf der Basis einer Entwicklersubstanz-Kupplersubstanz-Kombination, welches als Entwicklersubstanz ein Aminopyrazolderivat der allgemeinen Formel (I)

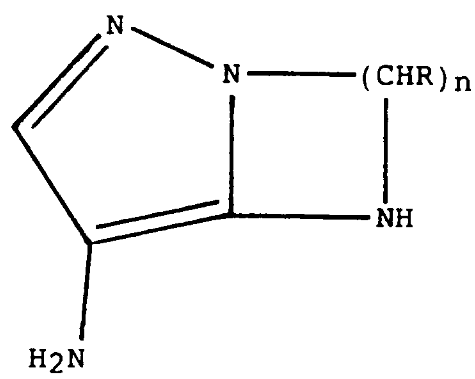

(I),

in der R = Wasserstoff oder ($C_1$ - $C_4$)-Alkyl bedeutet und n = 2 oder 3 ist oder dessen physiologisch verträgliche, wasserlösliche Salze enthält, die gestellte Aufgabe in hervorragender Weise gelöst wird.

In den Haarfärbemitteln sollen die Entwicklersubstanzen der Formel (I), von denen das 3-Amino-4,5,6,7-tetrahydro-pyrazolo[1,5-a]pyrimidin (R = H, n = 3), das 7-Amino-2,3-dihydro-1H-imidazo[1,2-b]pyrazol (R = H, n = 2) und das 3-Amino-6-methyl-4,5,6,7-tetrahydro-pyrazolo [1,5-a]pyrimidin (R = $CH_3$, n = 3) bevorzugt sind, in einer Menge von 0,01

bis 3,0 Gewichtsprozent, vorzugsweise in einer Menge von 0,1 bis 2,5 Gewichtsprozent, enthalten sein.

Obwohl die vorteilhaften Eigenschaften der hier beschriebenen neuen Entwicklersubstanzen es nahelegen, diese als alleinige Entwicklersubstanz zu verwenden, ist es selbstverständlich auch möglich, die Entwicklersubstanzen der Formel (I) gemeinsam mit bekannten Entwicklersubstanzen, wie zum Beispiel 1,4-Diaminobenzol, 2,5-Diaminotoluol oder 2,5-Diaminophenylethylalkohol, einzusetzen.

Als Kupplersubstanzen kommen als Bestandteil des hier beschriebenen Haarfärbemittels vorzugsweise Resorcin, 4-Chlorresorcin, 4,6-Dichlorresorcin, 2-Methylresorcin, 2-Amino-4-(2'-hydroxyethyl)amino-anisol, 2,4-Diaminobenzylalkohol, 2,4-Diaminophenylethylalkohol, m-Phenylendiamin, 2,4-Diamino-5-(2'-hydroxyethoxy)toluol, 5-Amino-2-methylphenol, 5-Amino-4-fluor-2-methylphenol, 5-Amino-2-ethyl-4-fluorphenol, 5-Amino-4-methoxy-2-methylphenol, 2,4-Diaminophenoxyethanol, 4-Amino-2-hydroxyphenoxyethanol, 1-Naphthol, 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1,2-methylendioxybenzol, 2,4-Diaminophenetol, 2,4-Diamino-5-methylphenetol, 4-Hydroxyindol, 3-Amino-5-hydroxy-2,6-dimethoxypyridin und 3,5-Diamino-2,6-dimethoxypyridin in Betracht.

Die Kuppler- und Entwicklersubstanzen können in dem Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,1 bis 5,0 Gewichtsprozent, wobei eine Menge von 0,5 bis 4,0 Gewichtsprozent bevorzugt ist.

Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhanden ist.

Weiterhin kann das erfindungsgemäße Haarfärbemittel zusätzlich andere Farbkomponenten, beispielsweise 6-Amino-2-methyl-phenol und 2-Amino-5-methylphenol, sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethyl)aminonitrobenzol und 2-Methylamino-5-bis(2'-hydroxyethyl)amino-nitrobenzol, Azofarbstoffe wie Acid Brown 4 (C.I. 14 805) und Dispersionsfarbstoffe wie beispielsweise 1,4-Diaminoanthrachinon und 1,4,5,8-Tetraaminoanthrachinon enthalten. Das Haarfärbemittel kann diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des neuen Haarfärbemittels kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol, Glycerin, oder Glykole wie 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, normal oder alkalisch reagieren. Insbesondere weist es einen pH-Wert von 8,0 bis 11,5 auf, wobei die Einstellung vorzugsweise mit Ammoniak erfolgt. Es können aber auch organische Amine, zum Beispiel Monoethanolamin und Triethanolamin, oder auch organische Basen wie Natriumhydroxid und Kaliumhydroxid Verwendung finden.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommen hauptsächlich Wasserstoffperoxid oder dessen Additionsverbindungen an Harnstoff, Melamin oder Natriumborat in Form einer 3- bis 12 prozentigen, vorzugsweise 6 prozentigen, wäßrigen Lösung in Betracht. Wird eine 6 prozentige Wasserstoffperoxid-Lösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 30 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die Herstellung der erfindungsgemäßen Entwicklersubstanzen ist in der Literatur noch nicht beschrieben.

Die Herstellung von 7-Nitro-2,3-dihydro-1H-imidazo-[1,2-b]pyrazol ist bei A. J. Goddard et al., Anti Cancer Drug Design 2, 235 (1987) beschrieben. Ausgangspunkt ist dort ein 5-Amino-4-cyano-1-(2'-hydroxyethyl)pyrazol, aus dem nach Ringschluß und anschließender Nitrierung die gewünschte Verbindung in einer Ausbeute von etwa 10 % erhalten wird.

Bessere Ausbeuten wurden dagegen durch Alkylierung mit Dibromalkanen und anschließende Reduktion der Nitrogruppe des bei H. Dorn et al., Liebigs-Ann. Chem. 707, (1967) 141 - 146 beschriebenen 3(5)-Amino-4-nitropyrazols erhalten.

Die erfindungsgemäßen Verbindungen lassen sich analog zu den vorstehend beschriebenen Verfahren herstellen.

Die Entwicklersubstanzen der Formel (I) sollen in dem Haarfärbemittel entweder als freie Basen oder in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden. Die Verbindungen der Formel (I) sind gut in Wasser löslich, sie weisen außerdem eine ausgezeichnete Lagerstabilität, insbesondere als Bestandteil der hier beschriebenen Haarfärbemittel auf.

Das erfindungsgemäße Haarfärbemittel mit einem Gehalt an Aminopyrazolderivaten als Entwicklersubstanz ermöglicht Haarfärbungen mit ausgezeichneter Farbechtheit, insbesondere was die Licht-, Wasch- und Reibechtheit anbetrifft, und die Haarfärbungen lassen sich mit Reduktionsmitteln wieder abziehen.

Hinsichtlich der färberischen Eigenschaften bieten die erfindungsgemäßen Haarfärbemittel Möglichkeiten, die weit über einen Ersatz der üblicherweise verwendeten 4-Aminophenole hinausgehen. So lassen sich brillante Rottöne mit außerordentlicher Farbtiefe erzeugen, wie sie mit den gängigen Farbkomponenten nicht zu erzielen sind. Neben dieser Anwendung im hochmodischen Bereich können aber auch durch die Verwendung in Kombination mit geeigneten Kupplungskomponenten natürliche Farbtöne erzeugt werden, ohne daß eine weitere Entwicklungskomponente vom Typ der p-Phenylendiamine erforderlich wäre.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Gegenstand der vorliegenden Patentanmeldung sind ferner neue Pyrazolderivate der allgemeinen Formel (II),

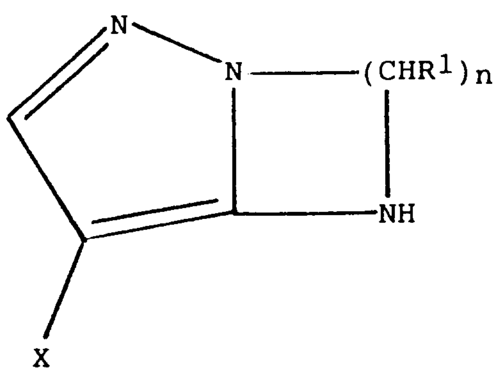

(II),

in der $R^1$ Wasserstoff oder ($C_1$- $C_4$)-Alkyl bedeutet, n gleich 2 oder 3 ist, wenn X eine Aminogruppe darstellt, und n gleich 3 ist, wenn X eine Nitrogruppe darstellt, wobei insbesondere 7-Amino-2,3-dihydro-1H-imidazo[1,2-b]pyrazol, 3-Nitro-4,5,6,7-tetrahydro-pyrazolo[1,5-a]pyrimidin, 3-Amino-4,5,6,7-tetrahydro-pyrazolo[1,5-a]pyrimidin, 3-Nitro-6-methyl-4,5,6,7-tetrahydro-pyrazolo[1,5-a]pyrimidin und 3-Amino-6-methyl-4,5,6,7-tetrahydro-pyrazolo[1,5-a]pyrimidin zu nennen sind.

In den nachstehenden Beispielen soll der Gegenstand der Erfindung näher erläutert werden.

## Herstellungsbeispiele

**Beispiel 1: Herstellung von 3-Amino-4,5,6,7-tetrahydro-pyrazolo[1,5-a]pyrimidin**

**Stufe 1: Synthese von 3-Nitro-4,5,6,7-tetrahydro-pyrazolo[1,5-a]pyrimidin**

Zu einer Suspension von 10 g (0,4 mol) Natriumhydrid in 200 ml getrocknetem DMF tropft man langsam eine Lösung von 51,2 g (0,4 mol) 3-Amino-4-nitro-pyrazol in 650 ml getrocknetem DMF. Nach Beendigung der Reaktion werden 121 g (0,6 mol) 1,3-Dibrompropan in 250 ml DMF zugegeben. Anschließend wird das Gemisch 5 Stunden lang unter Rückfluß erhitzt. Nach dem Abkühlen wird der Ansatz bis zur Trockene eingeengt. Beim Versetzen des Rückstandes mit 300 ml Dichlormethan und anschließendem Abkühlen auf 0 °C fällt das Produkt zusammen mit NaBr aus. Das Rohprodukt wird aus 1200 ml Ethanol umkristallisiert. Dabei werden 59 g (38 %) ockerfarbene Kristalle mit einem Schmelzpunkt von 251 - 252 °C erhalten.

$^1$H-NMR (DMSO-$d_6$, 60 MHz): δ = 7,89 (s, 1H, -NH, tauscht mit $D_2O$ aus), 7,85 (s, 1H, 3-H), 3,99 (t, 2H, J = 7 Hz, 1N-$C\underline{H_2}$-), 3,35 (mm, 2H, -NH-$\underline{CH_2}$-$CH_2$) und 2,03 ppm (m, 2H, -$CH_2$-$\underline{CH_2}$-$CH_2$).

MS (70 eV): m/3 = 168 ($M^+$)

**Stufe 2: Synthese von 3-Amino-4,5,6,7-tetrahydropyrazolo[1,5-a]pyrimidin**

0,6 g (3,57 mmol) 3-Nitro-4,5,6,7-tetrahydropyrazolo [1,5-a]pyrimidin werden in 130 ml Methanol mit 0,1 g Pd/C (10 prozentig) 2 Tage lang bei 25 °C und 50 bar hydriert. Nach dem Abfiltrieren des Katalysators wird das Filtrat mit methanolischer HCl versetzt und eingeengt. Das ausgefallene Produkt wird abgesaugt und mit wenig Methanol gewaschen. Die Verbindung liegt in Form des Trihydrochlorid-Hydrats, welches einen Schmelzpunkt von 179 - 182 °C aufweist, vor. Wird nach dem Abfiltrieren des Katalysators das Filtrat mit einer äquimolaren Menge Schwefelsäure versetzt, erhält man nach der Aufarbeitung die Verbindung in Form des Sulfats mit einem Schmelzpunkt von 176 - 179 °C.

$^1$H-NMR (DMSO-$d_6$, 60 MHz): δ = 9,50 (s, breit, 8H, N$\underline{H_2}$, $\underline{H}$Cl, $\underline{H_2}$O tauscht mit $D_2O$ aus), 7,75 (s, 1H, 3-H), 4,10 (t, 2H, J = 7 Hz, 1N-$\underline{C}H_2$), 3,29 (t, 2H, J = 7Hz, NH-$\underline{CH_2}$-$CH_2$) und 2,02 ppm (m, 2H, -$CH_2$-$\underline{CH_2}$-$CH_2$).

MS (70 eV): m/e = 138 ($M^+$)

**Beispiel 2: Herstellung von 7-Amino-2,3-dihydro-1H-imidazo[1,2-b]pyrazol-hydrosulfat**

0,15 g (0,97 mmol) 3-Nitro-2,3-dihydro-1H-imidazo[1,2-b] pyrazol werden wie in Beispiel 1, Stufe 2 hydriert. Nach dem Abfiltrieren des Katalysators wird eine äquimolare Menge Schwefelsäure zugegeben. Nach dem Einengen und Versetzen mit wenig Ethanol fällt das Produkt in Form beiger Kristalle mit einem Schmelzpunkt von 177 - 179 °C aus.

$^1$H-NMR (DMSO-$d_6$, 60 MHz): δ = 7,70 (s, breit 5H, NH, $NH_2$, $H_2SO_4$, tauscht mit $D_2O$ aus), 7,30 (s, 1H, 3-H) und 4,00 ppm (m, 4H,

MS (70 eV): m/e = 124 ($M^+$)

**Beispiel 3: Herstellung von 3-Amino-6-methyl-4,5,6,7-tetrahydropyrazolo[1,5-a]pyrimidin**

Stufe 1: Synthese von 3-Nitro-6-methyl-4,5,6,7-tetrahydropyrazolo[1,5-a]pyrimidin

Die Durchführung der Reaktion erfolgt analog Beispiel 1, Stufe 1 mit 1-Brom-3-chlor-2-methylpropan als Alkylierungsmittel. Man erhält gelbe Kristalle mit einem Schmelzpunkt von 190 - 192 °C in einer Ausbeute von 25 %.

1H-NMR(DMSO-$d_6$, 60mHz): δ = 7,92 (s,1H,-NH, tauscht mit $D_2O$ aus), 7,84 (s,1H, 2-CH), 4,07 (m,1H,N-$CH_2$-), 3,59 (m,1H,N-$CH_2$), 3,38 (m,1H,N-$CH_2$-), 2,98 (m,1H, NH-$CH_2$), 2,20 (m,1H,6C-H) und 1,02 ppm (d,3H,J=7$H_z$, 6C-$CH_3$).

MS (70 eV):m/e = 182 ($M^+$).

Stufe 2: Synthese von 3-Amino-6-methyl-4,5,6,8-tetrahydropyrazolo[1,5-a]pyrimidin-sulfat

Entsprechend Beispiel 1, Stufe 2 erhält man nach katalytischer Reduktion und Versetzen des Reaktionsgemisches mit Schwefelsäure das Sulfat mit einem Schmelzpunkt von 170 °C in Form farbloser Kristalle, in einer Ausbeute von 88 Prozent.

| | |
|---|---|
| 1H-NMR(DMSO-$d_6$, 60 $mH_z$): δ = | 7,40 ppm (s,5H,$NH_2$,NH, $H_2SO_4$ (tauscht mit $D_2O$ aus), 7,23 (s,1H, 2C-H), 4,25-1,80 (m, 5H,-$CH_2CH$-$CH_2$), 0,97 ppm (d,J=7$H_z$,6C-$CH_3$) |
| MS (70 eV):m/e = | 152 ($M^+$) |

**Beispiele für Haarfärbemittel**

**Beispiel 3: Haarfärbemittel in Gelform**

1,00 g 3-Amino-4,5,6,7-tetrahydro-pyrazolo[1,5-a] pyrimidin-sulfat
0,50 g 5-Amino-2-methylphenol
0,15 g Natriumsulfit, wasserfrei
5,00 g Laurylalkohol-diglykolethersulfat-Natriumsalz (28prozentige wäßrige Lösung)
1,00 g Hydroxyethylcellulose, hochviskos
10,00 g Ammoniak (22prozentige wäßrige Lösung)
82,35 g Wasser
100,00 g

50 g des vorstehenden Haarfärbemittels werden kurz vor dem Gebrauch mit 50 g Wasserstoffperoxidlösung (6prozentig) vermischt und das Gemisch anschließend auf blonde Naturhaare aufgetragen. Nach einer Einwirkungszeit von 30 Minuten bei 40 Grad Celsius wird das Haar mit Wasser gespült und getrocknet. Das Haar hat eine intensive purpurrote Färbung erhalten.

**Beispiel 5: Haarfärbemittel in Gelform**

0,55 g 3-Amino-2,3-dihydro-1H-imidazo[1,2-b]pyrazolhydrosulfat
0,27 g 3-Aminophenol
0,30 g Ascorbinsäure
15,00 g Ölsäure
7,00 g Isopropanol
10,00 g Ammoniak (22prozentige wäßrige Lösung)
66,88 g Wasser
100,00 g

Man vermischt kurz vor dem Gebrauch 50 g dieses Haarfärbemittels mit 50 g Wasserstoffperoxid-Lösung (6prozentig) und läßt ds Gemisch 30 Minuten lang bei 40 Grad Celsius auf weiße menschliche Haare einwirken. Sodann wird das Haar mit Wasser gespült und getrocknet. Das Haar ist in einem rot-violetten Farbton gefärbt.

**Beispiel 6: Färbemittel in Gelform**

1,05 g 3-Amino-4,5,6,7-tetrahydro-pyrazolo[1,5-a] pyrimidin-sulfat
0,80 g 2,5-Diaminotoluolsulfat
1,70 g 2-Amino-4-(2'hydroxyethyl)aminoanisolsulfat
0,10 g 1-(2'-Ureidoethyl)amino-4-nitrobenzol
0,15 g Natriumsulfit, wasserfrei
2,50 g Laurylalkohol-diglykolethersulfat-Natriumsalz (28prozentige wäßrige Lösung)
0,80 g Hydroxyethylcellulose, hochviskos
6,00 g Ammoniak (22prozentige wäßrige Lösung)
86.90 g Wasser
100,00 g

50 g des obigen Haarfärbemittels werden kurz vor dem Gebrauch mit 50 g Wasserstoffperoxid-Lösung (6prozentig) vermischt und die Mischung anschließend auf blonde Naturhaare aufgebracht. Nach einer Einwirkungszeit von 30

Minuten bei 40 Grad Celsius wird mit Wasser gespült und getrocknet. Das Haar hat eine aubergine Färbung erhalten.

**<u>Beispiel 7:</u> Haarfärbemittel in Gelform**

1,05 g 3-Amino-6-methyl-4,5,6,7-tetrahydro-pyrazolo[1,5-a]pyrimidin-sulfat
0,40 g 5-Amino-2-methylphenol
0,12 g 3-Aminophenol
0,15 g Natriumsulfit, wasserfrei
5,00 g Laurylalkohol-diglykolethersulfat-Natriumsalz (28prozentige wäßrige Lösung)
1,00 g Hydroxyethylcellulose, hochviskos
10,00 g Ammoniak (22prozentige wäßrige Lösung)
82,28 g Wasser
100,00 g

50 g des vorstehenden Haarfärbemitteis werden kurz vor dem Gebrauch mit 50 g Wasserstoffperoxidlösung (6prozentig) vermischt und das Gemisch anschließend auf blonde Naturhaare aufgetragen. Nach einer Einwirkungszeit von 30 Minuten bei 40 Grad Celsius wird das Haar mit Wasser gespült und getrocknet. Die Haare sind in einem intensiven Bordeaux-Ton gefärbt.

**Patentansprüche**

1. Mittel zur oxidativen Färbung von Haaren auf der Basis einer Entwicklersubstanz-Kupplersubstanz-Kombination, dadurch gekennzeichnet, daß es als Entwicklersubstanz ein Aminopyrazolderivat der allgemeinen Formel (I),

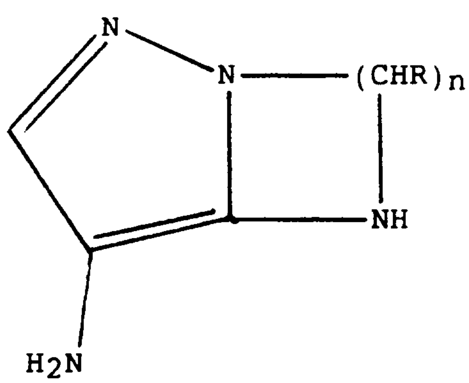

(I),

in der R = Wasserstoff oder ($C_1$ - $C_4$)-Alkyl bedeutet und n = 2 oder 3 ist, oder dessen physiologisch verträgliche wasserlösliche Salze enthält.

2. Mittel nach Anspruch 1, dadurch gekennzeichnet, daß das Aminopyrazol-Derivat der Formel (I) ausgewählt ist aus 3-Amino-4,5,6,7-tetrahydropyrazolo[1,5-a]pyrimidin, 7-Amino-2,3-dihydro-1H-imidazo[1,2-b]pyrazol oder 3-Amino-6-methyl-4,5,6,7-tetrahydro-pyrazolo[1,5-a]pyrimidin.

3. Mittel nach Anspruch 1 oder 2, dadurch gekennzeichnet, daß die Entwicklersubstanz der Formel (I) in einer Menge von 0,01 bis 3,0 Gewichtsprozent enthalten ist.

4. Mittel nach einem der Ansprüche 1 bis 3, dadurch gekennzeichnet, daß die Kupplersubstanz ausgewählt ist aus Resorcin, 4-Chlorresorcin, 4,6-Dichlorresorcin, 2-Methylresorcin, 2-Amino-4-(2'-hydroxyethyl)amino-anisol, 2,4-Diaminobenzylalkohol, 2,4-Diaminophenylethylalkohol, m-Phenylendiamin, 2,4-Diamino-5-(2'-hydroxyethoxy)toluol, 5-Amino-2-methylphenol, 5-Amino-4-fluor-2-methylphenol, 5-Amino-2-ethyl-4-fluorphenol, 5-Amino-4-methoxy-2-methylphenol, 2,4-Diaminophenoxyethanol, 4-Amino-2-hydroxyphenoxyethanol, 1-Naphthol, 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1,2-methylendioxybenzol, 2,4-Diaminophenetol, 2,4-Diamino-5-methylphenetol, 4-Hydroxyindol, 3-Amino-5-hydroxy-2,6-dimethoxypyridin und 3,5-Diamino-2,6-dimethoxypyridin.

5. Mittel nach einem der Ansprüche 1 bis 4, dadurch gekennzeichnet, daß die Gesamtmenge der Entwickler-Kupplersubstanz-Kombination 0,1 bis 5,0 Gewichtsprozent, vorzugsweise 0,5 bis 4,0 Gewichtsprozent, beträgt.

6. Mittel nach einem der Ansprüche 1 bis 5, dadurch gekennzeichnet, daß es eine Farbkomponente enthält, die ausgewählt ist aus 6-Amino-2-methylphenol, 2-Amino-5-methylphenol, Diamond Fuchsine (C.I. 42 510), Leather Ruby HF (C.I. 42 520), 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethyl)amino-nitrobenzol, 2-Methylamino-5-bis(2'-hydroxyethyl)amino-nitrobenzol, Acid Brown 4 (C.I. 14 805), 1,4-Diaminoanthrachinon und 1,4,5,8-Tetraaminoanthrachinon.

7. Pyrazolderivat der allgemeinen Formel (II),

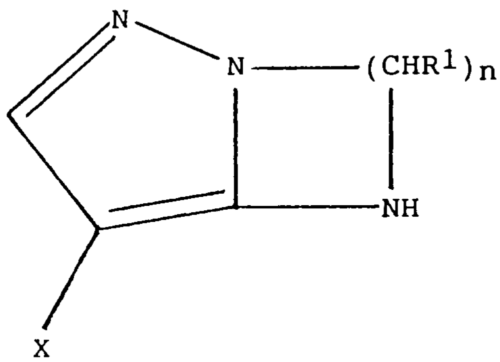

(II),

in der $R^1$ Wasserstoff oder ($C_1$- $C_4$)-Alkyl bedeutet, n gleich 2 oder 3 ist, wenn X eine Aminogruppe darstellt, und n gleich 3 ist, wenn X eine Nitrogruppe darstellt.

8. 7-Amino-2,3-dihydro-1H-imidazo[1,2-b]pyrazol

9. 3-Nitro-4,5,6,7-tetrahydro-pyrazolo[1,5-a] pyrimidin

10. 3-Amino-4,5,6,7-tetrahydro-pyrazolo[1,5-a] pyramidin

11. 3-Nitro-6-methyl-4,5,6,7-tetrahydro-pyrazolo [1,5-a]pyrimidin

12. 3-Amino-6-methyl-4,5,6,7-tetrahydro-pyrazolo [1,5-a]pyrimidin

## Claims

1. Oxidative hair-colouring agent based on a developer-coupler combination, characterized in that it comprises, as the developer, an aminopyrazole derivative of the general formula (I)

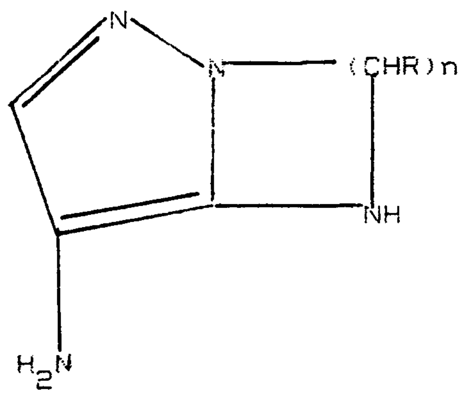

(I),

in which R stands for hydrogen or a ($C_1$-$C_4$) alkyl group and n is 2 or 3, or physiologically well-tolerated water-soluble salts thereof.

2. Agent according to Claim 1, characterized in that the aminopyrazole derivative of formula (I) is selected from 3-amino-4,5,6,7-tetrahydro-pyrazolo[1,5-a]pyrimidine, 7-amino-2,3-dihydro-1H-imidazo[1,2-b]pyrazole or 3-amino-6-methyl-4,5,6,7-tetrahydropyrazolo[1,5-a]pyrimidine.

3. Agent according to Claim 1 or Claim 2, characterized in that it comprises a quantity of from 0.01 to 3.0 per cent by weight of the developer of formula (I).

4. Agent according to any one of claims 1 to 3, characterized in that the coupler is selected from resorcinol, 4-chlororesorcinol, 4,6-dichlororesorcinol, 2-methylresorcinol, 2-amino-4-(2'-hydroxyethyl)amino-anisole, 2,4-diaminobenzyl alcohol, 2,4-diaminophenylethyl alcohol, m-phenylenediamine, 2,4-diamino-5-(2'-hydroxyethoxy)toluol, 5-amino-2-methylphenol, 5-amino-4-fluoro-2-methylphenol, 5-amino-2-ethyl-4-fluorophenol, 5-amino-4-methoxy-2-methylphenol, 2,4-diaminophenoxyethanol, 4-amino-2-hydroxyphenoxyethanol, 1-naphthol, 3-aminophenol, 3-amino-2-methylphenol, 4-hydroxy-1,2-methylenedioxybenzene, 4-amino-1,2-methylenedioxybenzene, 4-(2'-hydroxyethyl)amino-1,2-methylenedioxybenxene, 2,4-diaminophenetole, 2,4-diamino-5-methylphenetole, 4-hydroxyindole, 3-amino-5-hydroxy-2,6-dimethoxypyridine and 3,5-diamino-2,6-dimethoxypyridine.

5. Agent according to any one of Claims 1 to 4, characterized in that the total quantity of the developer-coupler combination is from 0.1 to 5.0 per cent by weight, preferably from 0.5 to 4.0 per cent by weight.

6. Agent according to any one of Claims 1 to 5, characterized in that it comprises a dye component selected from 6-amino-2-methylphenol, 2-amino-5-methylphenol, diamond fuchsine (C.I. 42 510), leather ruby HF (C.I. 42 520), 2-nitro-1,4-diaminobenzene, 2-amino-4-nitrophenol, 2-amino-5-nitrophenol, 2-amino-4,6-dinitrophenol, 2-amino-5-(2'-hydroxyethyl)aminonitrobenzene, 2-methylamino-5-bis(2'-hydroxyethyl)aminonitrobenzene, acid brown 4 (C.I. 14 805), 1,4-diaminoanthraquinone and 1,4,5,8-tetra-aminoanthraquinone.

7. Pyrazole derivative of the general formula (II),

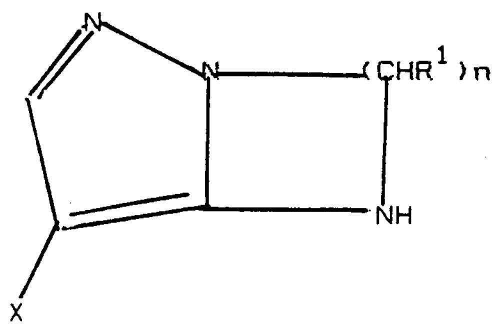

(II),

in which $R^1$ stands for hydrogen or a ($C_1$-$C_4$) alkyl group, n is 2 or 3 and X represents an amino-group, and n is 3 when X represents a nitro group.

8. 7-amino-2,3-dihydro-1H-imidazo[1,2-b]pyrazole.

9. 3-nitro-4,5,6,7-tetrahydro-pyrazolo[1,5-a]pyrimidine.

10. 3-amino-4,5,6,7-tetrahydro-pyrazolo[1,5-a]pyrimidine.

11. 3-nitro-6-methyl-4,5,6,7-tetrahydro-pyrazolo[1,5-a]pyrimidine.

12. 3-amino-6-methyl-4,5,6,7-tetrahydro-pyrazolo[1,5-a]pyrimidine.

**Revendications**

1. Agent de teinture oxydante de cheveux, à base d'une combinaison substance révélatrice-substance de couplage, caractérisé en ce que la substance révélatrice utilisée est un dérivé d'aminopyrazole de formule générale (I),

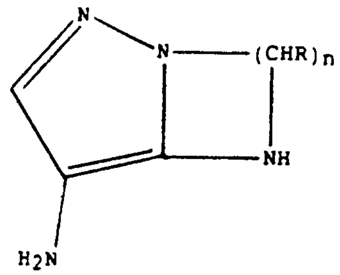

(I),

dans laquelle R = hydrogène ou un alkyl en ($C_1$ à $C_4$) et n = 2 ou 3, ou contient ses sels solubles dans l'eau, compatibles physiologiquement.

**2.** Agent selon la revendication 1, caractérisé en ce que le dérivé d'aminopyrazole de formule (I) est sélectioné parmi la 3-amino-4,5,6,7-tétrahydropyrazolo [1,5-a] pyrimidine, le 7-amino-2,3-dihydro-1H-imidazo[1,2-b]pyrazole ou la 3-amino-6-méthyl-4,5,6,7-tétrahydro-pyrazolo [1,5-a] pyrimidine.

**3.** Agent selon la revendication 1 ou 2, caractérisé en ce que la substance révélatrice de formule (I) est contenue en une quantité allant de 0,01 à 3,0 % en poids.

**4.** Agent selon l'une des revendications 1 à 3, caractérisé en ce que la substance de couplage est sélectionée parmi la résorcine, la 4-chlororésorcine, la 4,6-dichlororrésorcine, la 2-méthytrésorcine, le 2-amino-4-(2'-hydroxyéthyl)amino-anisol, l'alcool 2,4-diaminobenzylique, l'alcool 2,4-diaminophényléthylique, la m-phénylènediamine, le 2,4-diamino-5-(2'-hydroxyéthoxy)toluol, le 5-amino-2-méthylphénol, le 5-amino-4-fluor-2-méthylphénol, le 5-amino-2-éthyl-4-fluorophénol, le 5-amino-4-méthoxy-2-méthylphénol, le 2,4-diaminophénoxyéthanol, le 4-amino-2-hydroxyphénoxyéthanol, la 1-naphtol, le 3-aminophénol, le 3-amino-2-méthylphénol, le 4-hydroxy-1,2-méthylènedioxybenzène, le 4-amino-1,2-méthlènedioxybenzène, le 4-(2'-hydroxyéthyl)amino-1,2-méthlènediaoxybenzène, le 2,4-diaminophénétole, le 2,4-diamino-5-méthyiphénétole, le 4-hydroxyindole, la 3-amino-5-hydroxy-2,6-diméthoxypyridine et la 3,5-diamino-2,6-diméthoxypyridine.

**5.** Agent selon l'une des revendications 1 à 4, caractérisé en ce que la quantié totale de combinaison substance révélatrice-substance de couplage est de 0,1 à 5,0 % en poids, de préférence de 0,5 à 4,0 % en poids.

**6.** Agent selon l'une des revendications 1 à 5 caractérisé en ce qu'il contient un colorant, sélectionné parmi : le 6-amino-2-méthylphénol, le 2-amino-5-méthylphénol, Diamond Fuchsine (C.I. 42 510), Leather Ruby HF (C.I. 42 520), le 2-nitro-1,4-diaminobenzène, le 2-amino-4-nitrophénol, le 2-amino-5-nitrophénol, le 2-amino-4,6-dinitrophénol, le 2-amino-5-(2'-hydroxyéthyl)amino-nitrobenzène, le 2-méthylamino-5-bis(2'-hydroxyéthyl)amino-nitrobenzène, l'Acid Brown 4 (C.I. 14 805), la 1,4-diaminoantrachinone et la 1,4,5,8-tétraaminoantrachinone.

**7.** Dérivé de pyrazole de formule générale (II),

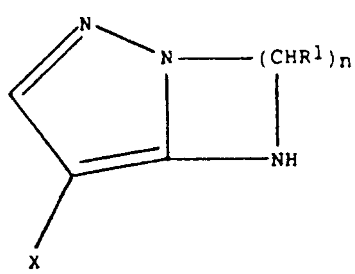

(II),

dans laquelle $R^1$ = hydrogène ou un alkyl en ($C_1$ à $C_4$) et n = 2 ou 3 si X constitue un groupe amino ou n = 3 Si X constitue un group nitro.

**8.** 7-amino-2,3-dihydro-1H-imidazo[1,2-b]pyrazole.

**9.** 3-nitro-4,5,6,7-tétrahydro-pyrazolo[1,5-a] pyrimidine.

**10.** 3-amino-4,5,6,7-tétrahydro-pyrazolo[1,5-a] pyrimidine.

**11.** 3-nitro-6-méthyl-4,5,6,7-tétrahydro-pyrazolo[1,5-a] pyrimidine.

**12.** 3-amino-6-méthyl-4,5,6,7-tétrahydro-pyrazolo[1,5-a] pyrimidine.